# EUROPEAN PATENT APPLICATION

(11) **EP 1 911 441 A2**
(43) Date of publication of application: **16.04.2008**
(21) Application number: 07019437.8
(22) Date of filing: 04.10.2007
(51) Int. Cl.: A61K 9/00, A61K 9/16, A61K 9/20, A61K 9/48, A61K 31/405

(54) **Controlled release color stable pharmaceutical dosage form of HMG-COA reductase inhibitors, free of alkalizing or buffering agents**

(30) Priority: 11.10.2006 IN KO10462006
(71) Applicant: Lupin Limited, Mumbai 400 098, Maharashtra (IN)
(72) Inventor: Mistry, Dhanashree, B., Pune 411 042 Maharashtra (IN); Panigrahi, Dhananjay, Pune 411 042 Maharashtra (IN); Kumar, T., Vijaya, Pune 411 042 Maharashtra (IN); Sen, Himadri, Pune 411 042 Maharashtra (IN)
(74) Representative: Wibbelmann, Jobst

(57) **Abstract**

A color stable controlled release pharmaceutical dosage form comprising HMG CoA reductase inhibitor, rate controlling polymers and one or more pharmaceutical excipients wherein the granules are independent of particle size.the pharmaceutical dosage form is free of alkalizing/buffering agents. A color stable controlled release pharmaceutical dosage form comprising Fluvastatin Sodium, hydroxypropyl methylcellulose polymer and hydroxypropyl cellulose, wherein the granules are independent of particle size.

## Description

### FIELD OF THE INVENTION

The present invention relates to a controlled release color stable pharmaceutical dosage forms comprising the active ingredient and other excipients, free of alkalizing or buffering agents.

More particularly the present invention relates to controlled release color stable pharmaceutical dosage wherein the active substance is selected from the group of HMG-CoA reductase inhibitors like statins, most preferably the statin is Fluvastatin and its salts.

The present invention also relates to the methods for the preparation of controlled release color stable pharmaceutical dosage forms comprising HMG-Co A reductase inhibitor free of alkalizing or buffering agents wherein the granules are independent of particle size.

### BACKGROUND AND PRIOR ART

US 5,356,896 disclose pharmaceutical composition containing fluvastatin and alkalizing substance capable of imparting a PH of at least 8 to the formulation.

US 6,558,659 discloses stable pharmaceutical dosage forms containing a ring-opened 7-substituted-3, 5-dihydroxyheptanoic or a ring-opened 7-substituted-3, 5- dihydroxyheptenoic acid, or a pharmaceutically acceptable salt thereof, as an active component and a stabilizing effective amount of at least one amido-group containing polymeric compound or at least one amino-group containing polymeric compound, or combination thereof; wherein the stabilized pharmaceutical composition does not contain a stabilizing effective amount of another stabilizer or a combination of other stabilizers.

WO 02/076376 discloses stabilization of pravastatin with carriers, which comprise at least one diluent and at least one lubricant.

EP 336298**,** US 5,030,447 and US 5,180,589, respectively, disclose stabilization of pravastatin with basifying substances, selected from, strong bases such as hydroxides of alkali metals and alkaline earth metals, and ammonium hydroxide, preferably specifying the substances such as magnesium oxide, magnesium hydroxide, calcium hydroxide sodium hydroxide, potassium hydroxide and lithium hydroxide, and ammonium hydroxide and magaldrate.

WO 2000/35425 discloses stabilization of the statins with buffers, among which are listed, for example sodium and potassium citrate, sodium phosphate, disodium phosphate, calcium carbonate, calcium hydrogen phosphate, calcium phosphate, calcium sulfate, sodium or magnesium carbonate, sodium ascorbinate, benzoate, sodium or potassium hydrogen carbonate, sodium or potassium lauryl sulfate and mixtures thereof.

WO 2001/93860 discloses stabilization of statins with co-crystallization and/or co- precipitation of a statin and a buffering or basifying substance in a homogeneous mixture of statins and said buffering or basifying substance. The following buffering substances are described, for example sodium and potassium citrate, sodium and potassium phosphate or hydrogen phosphate, dibasic sodium phosphate, sodium, potassium, magnesium or calcium carbonate or hydrogen carbonate, sulfates, aminoguanidine carbonate or hydrogen carbonate, guanidine carbonate or hydrogen carbonate, succinimide carbonate or hydrogen carbonate, 1-adamantil amine carbonate or hydrogen carbonate etc. The following basifying substances are described, for example metallic oxides such as magnesium oxide, aluminium oxide, hydroxides of alkaline and alkaline earth metals and organic bases such as succinimide, 1-adamantil amine, N, N'-bis (2hydroxyethyl) ethylenediamine, tris (hydroxymethyl) aminomethane, D (-)-N-methylglucamine and organic acids with alkaline character such as 3- (N-morpholino) propanesulfonic acid, 4-(cyclohexyl amino)-1-butanesulfonic acid, 4-(cyclohexyl amino)-1-ethansulfonic acid and salts of alkali and alkaline earth metals with said acids or with arginine, ornithine, lysine, etc.

EP 547000 and US 5,356,896 respectively, discloses stabilization of fluvastatin with an alkaline medium which is either an alkali or a buffer and specifies water-soluble alkaline substances from the group consisting of inorganic carbonate salts such as sodium or potassium carbonate, sodium bicarbonate, potassium hydrogen carbonate, phosphate salts such as anhydrous sodium, potassium or calcium dibasic phosphate, trisodium phosphate and hydroxides of alkaline metals such as sodium, potassium or lithium hydroxide and mixtures thereof. Further it also specifies water insoluble or low soluble alkaline substances such as magnesium oxide, hydroxide or carbonate, magnesium hydrogen carbonate, aluminium or calcium hydroxide and carbonate, combined compounds of aluminum and magnesium such as magnesium aluminium hydroxide, and the phosphoric acid salts such as tribasic calcium phosphate and mixtures thereof.

WO 03/000239 describes stabilization of pravastatin with buffers such as, e.g. TRIS trometamine and disodium hydrogen phosphate.

US 6,242,003 describes a color stable sustained release tablet comprising granules comprising fluvastatin and a hydroxypropyl methyl cellulose polymer; wherein the granules have a mean particle size of less than 200 micron. Additionally it also contains hydrophilic polymers like hydroxy propyl cellulose.

It was seen that dosage forms comprising HPMC polymers formed gel-like domains upon storage, these domains, still more surprisingly were highly coloured. Whereas this discolouration left the dosage forms with an unsightly, uneven mottled appearance. It was believed that the increased colour stability observed as the mean particle size is decreased may be due to the ability of the finer granules to form a tighter compact when compressed, thereby reducing the incidence and size of any voids in the compacted mass. As the HPMC gels are thought to form in these voids, the smaller their number and/or size, the less tendency there is for gels to form.

US 2003/0171419 discloses composition comprising fluvastatin and HPMC, which is colour-stable upon prolonged periods of storage. Also it describes granules comprising fluvastatin and HPMC wherein the granules have a mean particle size of less than 200 microns. Further it describes a method of preventing or substantially reducing the formation of gels of hydrated HPMC in a pharmaceutical composition or in granules comprising a fluvastatin and HPMC comprising the step of storing the pharmaceutical composition or granules at a relative humidity not exceeding 75% and at a temperature of between 25°C and 40°C.

While a number of controlled releases stable formulations comprising fluvastatin sodium with alkalizing and buffering agents are available but there remains a need for stable formulations free of alkalizing and buffering agents. Another drawback is that formulations of the prior art use granules comprising fluvastatin and rate controlling polymer having a specific particle size. However, the formulations of the present invention are developed free of alkalizing or buffering agent wherein the granules are independent of particle size manufactured under normal atmospheric conditions.

### OBJECTIVE OF THE INVENTION

Accordingly one object of the present invention is to provide a color stable controlled release pharmaceutical dosage form comprising HMG CoA reductase inhibitor, hydroxypropyl methyl cellulose, hydroxypropyl cellulose free of alkalizing, buffering agents and wherein the granules are independent of particle size.

According to another aspect, a color stable controlled release pharmaceutical dosage form comprising granules comprising fluvastatin sodium and a hydroxypropyl cellulose polymer, free of alkalizing, buffering agents, wherein the granules are independent of particle size.

Another object of the present invention is to provide a process for the preparation of controlled release formulation of Fluvastatin sodium.

### SUMMARY OF INVENTION

According one aspect of the present invention there is provided color stable controlled release pharmaceutical dosage comprising of a drug like HMG-Co A reductase inhibitor, hydroxypropyl cellulose, rate controlling polymers and one or more pharmaceutical excipients and free of alkalizing or buffering agents or combinations thereof, wherein the granules are independent of particle size.

According to another aspect, the invention provides A process of making a color stable controlled release dosage form comprising fluvastatin sodium and hydroxypropyl cellulose wherein the drug and excipients granulation is carried out under dry conditions and is capable of being stored at normal atmospheric conditions.

### DETAILED DESCRIPTION

The present invention is concerned with formulations for controlled release of fluvastatin, free of alkalizing or buffering agents that are independent of particle size of granules.

The present invention relates to a color stable controlled release pharmaceutical dosages form comprising Fluvastatin, and one or more pharmaceutical excipients, free of alkalizing or buffering agents and which is independent of particle size of granules.

While refereeing to the prior art it is observed that formulation comprising fluvastatin and hydrophilic polymers with stabilizers imparts stable formulation. However surprisingly it was found that present invention comprising fluvastatin and hydrophilic polymers free of alkalizing and/ or buffering agents wherein the granules are independent of particle size.

HPC (Hydroxypropyl cellulose) has been used in drug formulations, but generally only in combinations with other materials, polymers to provide a controlled release.

Surprisingly it has been found that compositions comprising, Fluvastatin and hydroxypropyl cellulose of various viscosity grades, provides a stable controlled release formulation, which unlike the prior art is independent of particle size of the granules used. Also these dosage forms do not exhibit the mottled appearance as seen with the prior art dosage form upon prolonged storage at normal atmospheric condition.

Though it has been seen that amorphous form of fluvastatin is less stable than crystalline form but interestingly it is observed that during manufacturing process limiting the moisture content can prevent the conversion of amorphous from to crystalline form of fluvastatin.

Surprisingly other hydrophilic polymers such as hydroxy propyl cellulose result in stable dosage forms, which do not depend upon the particle size of the granules

Rate controlling polymer according to present invention includes agents, which controls the release of drug from the formulation. The agents comprise of hydroxy propyl methylcellulose in the range of less than about 15%, and other hydrophilic polymer as described herein.

Hydrophilic polymers that may be used in the composition are selected from the group comprising of hydroxyethyl cellulose having a number average molecular weight ranging from 90,000 to 1,300,000, hydroxypropylcellulose having a number average molecular weight of 80,000 to 1,150,000 in the range of less than about 50 % preferably about 15% to about 50% and poly (ethylene oxide) having a number average molecular weight ranging from 100,000 to 500,000, preferably 150,000 to 300,000, more preferably 200,000.

The scope of the present invention is not limited to the molecular weights of the rate controlling hydrophilic polymers.

The stable controlled release pharmaceutical dosage form wherein the ratio of the rate controlling polymer hydroxypropyl methylcellulose to hydroxy propylcellulose is from about 1:3.5 to about 1:10.

Many active substances are sensitive to humidity. At increased humidity water is bound to the active substance itself and/or pharmaceutical excipients surrounding the active substance which associated with one or more other environmental influences may thus trigger degradation reactions of the active substance. It is known from the prior art that HGM-CoA reductase inhibitors, e.g. Fluvastatin, pravastatin and atorvastatin are also sensitive to humidity.

It is surprisingly found that during manufacturing process as disclosed in prior art using amorphous active pharmaceutical ingredient preferably fluvastatin sodium with a moisture content in the range of about 4% to about 6%, preferably about 5% there is no conversion of amorphous form to crystalline form.

Also it has been found that Test formulation having a LOD content in a range of 5.46 -5.51 is stable and there is no conversion of amorphous form to crystalline form. Refer to table 1 below.

**Table: 1 Loss on Drying (LOD) Data**

| **Batch Details** | **LOD at 105°, under vacuum at 105°C for 3 Hrs. (%w/w)** |
|---|---|
| Innovator: Lescol XL | 9.28 |
| Test formulation | 5.49 |
| Test formulation | 5.46 |
| Test formulation | 5.51 |

Loss of drying (LOD) test is carried out in dry air oven under vacuum at 105° till constant weight.

The pharmaceutical excipients of the color stable controlled release pharmaceutical dosage forms of the present invention are selected from the group comprise of different types of cellulose like hydroxypropyl cellulose.

Controlled release formulation describes a formulation that does not release active drug substance immediately after oral dosing and that allows a reduction in dosage frequency. A controlled release formulation includes but is not limited to extended release, delayed release, sustained release formulations. A controlled release formulation may be administered once a day. Rate controlling polymer according to present invention includes agents, which controls the release of drug from the formulation.

Further the controlled release formulations of the present invention are manufactured under normal atmospheric conditions.

The normal atmospheric conditions under which the tablets are generally manufactured are at a temperature not more than about 25°C and relative humidity of about 40%.

An example of controlled release formulation is matrix formulation (erodable and non-erodable), diffusion controlled membrane coated or osmotic pumps.

The color stable controlled release pharmaceutical dosage form comprises of HMG CoA reductase inhibitors like the statins, for example, the following are known: pravastatin, atorvastatin, simvastatin, lovastatin, mevastatin or compactin, fluvastatin, cerivastatin or rivastatin, rosuvastatin or visastatin, and itavastatin or pitavastatin, or nisvastatin and its pharmaceutically acceptable salts.

US 5356896 discloses a color stable composition comprising a drug and other pharmaceutically acceptable excipients imparting a pH of atleast 8. The present scope of the invention comprises of a drug, and one or more pharmaceutical excipients and does not contain alkalizing or buffering substances or combinations thereof, imparting a pH of less than 10.

Preferred pharmaceutical compositions are tablets, which are formed of granules having a mean granule particle size of less than about 600 micron.
The particle size analysis for the present invention is carried out using Malvern Method.
However, with no more than routine experimentation, the skilled person will be able to determine a suitable granulate size for a given excipients mixture.
Granules described as herein above comprise of Fluvastatin sodium, rate controlling polymer and one or more pharmaceutical excipients.

**Table: 2 Particle size Analysis Data using Malvern Method**

| Solvent used: Light liquid paraffin | | | |
|---|---|---|---|
| **Test Sample** | | | |
| **Sample** | **Particle Size in µm** | | |
| | **d(0.1)** | **d(0.5)** | **d(0.9)** |
| **Average (n= 3)** | 94.03 | 313.27 | 628.60 |
| **SD** | 4.77 | 18.04 | 61.23 |
| **%RSD** | 5.07 | 5.76 | 9.74 |

The formulation will, in general comprise of one or more excipients. Examples of excipients include, but are not limited to, diluents, disintegrants, lubricant, glident, binders, fillers, surfactant, solubilizers, and wetting agents. A combination of excipients may also be used.

Such excipients include diluents such as mannitol, dextrose, xylitol, sorbitol, gelatin, acacia, sucrose, microcrystalline cellulose, calcium carbonate, calcium phosphate dibasic, calcium phosphate tribasic, calcium sulfate, lactose, starches, vinyl polymers and the likes, disintegrants referred to in the present invention include one or more of microcrystalline cellulose, croscarmellose sodium, crospovidone, carboxymethyl starch sodium, sodium starch glycolate and the likes, binders referred to in the present invention include one or more celluloses such as hydroxypropyl cellulose, hydroxy ethyl cellulose, ethyl cellulose, hydroxypropyl methyl cellulose, methyl cellulose or mixtures thereof, acrylates, methacrylates, povidone, sucrose, corn or maize starch, pregelatinized starch and the like, coloring agents such as ferric oxide, FD&C dyes, lakes and the likes and flavoring agent. Examples of glidents include but are not limited to silica, magnesium trisilicate, powdered cellulose, talc, and starch.

Further one or more excipients may optionally contain alkalizing or buffering agents and or antioxidants known to a person skilled in the art.

Other components for solid pharmaceutical dosage forms are the components, which are known and are conventional in the prior art and are used for solid pharmaceutical dosage forms in the said art.

The formulation of the present invention may be prepared by conventional techniques well known to those skilled in the art such as wet granulation, Non- aqueous granulation, melt granulation, direct compression or dry compaction (slugging) and the like. Most preferably the formulation of the present invention is prepared by dry compaction and or slugging.

A process of making a color stable controlled release dosage form comprising fluvastatin and hydroxypropyl cellulose wherein the drug and excipients granulation is carried out under dry conditions, wherein the dry condition is direct compaction or dry compaction (slugging) carried under atmospheric condition.

The above mentioned stable controlled release pharmaceutical dosage form is capable of being stored at normal atmospheric conditions.

Most preferably the formulation and/ or dosage form of the present invention may be a tablet or granules filled in capsule or pellet. Said dosage form may be uncoated or coated.

The active substance when required can be coated with film coating which of said invention and provides protection of the active substance from the environmental factors, or coated with any other in the prior art known film coating.

The dosage forms may be coated with one or more coatings, enteric or film coating as is well known in the art such as shellac, zein, hydroxypropylmethylcellulose, ethyl cellulose, hydroxy propyl cellulose, polymethacrylates, polyvinyl acetate pthalate, cellulose acetate pthalate, triacetin, dibulty sebecate, a mixture of polyethylene glycol, titanium dioxide and the like.

The coating may be performed by conventional means using commercially available, ready-to-coat preparations, sold under various brand names such as various grades of Opadry®, Dispersions or mixtures thereof and the like. Preferably hydroxy propyl cellulose used in the coating solution is in the range of about 30% to about 40%, Talc in the range of about 12% to about 25%, PEG 6000 in the range of about 10% to about 20% and Titanium dioxide in the range of about 14% to about 33%.

Further it was found that during the coating process upon use of a hydroalcholic dispersion of opadry in Isopropyl alcohol (about 75 %) and water (about 25%) the conversion of amorphous form to crystalline form was minimized.

The dissolution of the present invention was carried out in type 1 dissolution apparatus, basket, at 50rpm, at a temperature of 37±0.5°C, in 1000ml of water and may release not more than about 50% of Fluvastatin is released after 2 hours of measurement in said apparatus, from about 50% to about 80% of Fluvastatin is released after 4 hours of measurement in said apparatus, not less than about 80 % of Fluvastatin is released after 8 hours of measurement in said apparatus.

The following examples are illustrative of the present invention, and the example should not be considered as limiting the scope of this invention in any way, as these examples and other equivalents thereof will become apparent to those versed in the art, in the light of the present disclosure, and the accompanying claims.

### Example 1

| **Ingredients** | **% W/w** |
|---|---|
| *Intra -granular* | |
| Fluvastatin Sodium | 26 |
| Avicel PH 112 | 22 |
| Aerosil 200 | 4 |
| HPMC K100M CR Premium | 8 |
| HPC | 27 |
| Magnesium Stearate | 1 |
| *Extra-granular* | |
| Avicel PH 112 | 7 |
| Aerosil 200 | 1 |
| Magnesium Stearate | 1 |
| Film Coating | |
| **Opadry White** contains: | 4.00 |
| HPC | |
| Talc | |
| Titanium Dioxide | |
| Macrogol (PEG 6000) | |

### Example 2

| **Ingredients** | **% W/w** |
|---|---|
| Simvastatin | 26 |
| Avicel PH 112 | 20 |
| Aerosil 200 | 4 |
| HPMC K100M CR Premium | 10 |
| HPC | 30 |
| Magnesium Stearate | 2 |
| *Extra-granular* | |
| Avicel PH 112 | 7 |
| Aerosil 200 | 1 |
| Magnesium Stearate | 1 |
| Film Coating | |
| **Opadry White** contains: | 4.00 |
| HPC | |
| Talc | |
| Titanium Dioxide | |
| Macrogol (PEG 6000) | |

### Example 3:

| **Ingredients** | **% W/w** |
|---|---|
| *Intra -granular* | |
| Fluvastatin Sodium | 26 |
| Avicel PH 112 | 25 |
| Perlitol 200 | 5 |
| Aerosil 200 | 5 |
| HPC | 14 |
| HPC | 15 |
| Magnesium Stearate | 2 |
| *Extra-granular* | |
| Avicel PH 112 | 8 |
| Aerosil 200 | 2 |
| Magnesium Stearate | 1.5 |
| Film Coating | |
| **Opadry White** contains: | 4.00 |
| HPC | |
| Talc | |
| Titanium Dioxide | |
| Macrogol (PEG 6000) | |

### Example 4:

| **Ingredients** | **% W/w** |
|---|---|
| *Intra -granular* | |
| Pitvastatin | 26 |
| Avicel PH 112 | 30 |
| Aerosil 200 | 5 |
| HPC | 13 |
| HPC | 15 |
| Magnesium Stearate | 2 |
| *Extra-granular* | |
| Avicel PH 112 | 8 |
| Aerosil 200 | 2 |
| Magnesium Stearate | 2 |
| Film Coating | |
| **Opadry White** contains: | 4.00 |
| HPC | |
| Talc | |
| Titanium Dioxide | |
| Macrogol (PEG 6000) | |

### Brief manufacturing procedure:

a. Mix and sift the weighed quantities of active agent (drug) and other intragranular excipients except Magnesium stearate for sufficient time.
b. Lubricate the blend with the weighed quantity of Magnesium stearate for sufficient time.
c. Compact the blend
d. Mill the step c. slugs in order to pass through 25# sieve and separate the oversized and undersized granules by using 50# sieve. Perform reslugging of the undersized granules till the granular portion NLT 80% is retained on 50# sieve. Finally sift all the granules through 25# sieve.
e. Lubricate the step d. blend with the extra granular excipients, compressed and coated.

### Table 3: Dissolution Profile

Table 3 provides the comparative dissolution profile of stable controlled release compositions of Fluvastatin sodium of the present invention and marketed Lescol XL.

| **Time in Hr.** | **AVg. % Cum. Release** | |
|---|---|---|
| | **Lescol XL** | **Test Sample** |
| 0.5 | 6.0 | 6.0 |
| 1 | 12 | 12 |
| 2 | 24 | 28 |
| 4 | 56 | 59 |
| 6 | 85 | 85 |
| 8 | 102 | 100 |

The formulation of the present invention, as in table 2, may release not more than about 50% of Fluvastatin is released after 2 hours of measurement in said apparatus, from about 50% to about 80% of Fluvastatin is released after 4 hours of measurement in said apparatus, not less than about 80 % of Fluvastatin is released after 8 hours.

### Table 4: Stability Data

Table 4 provides Stability data of controlled release compositions of Fluvastatin sodium of the present invention when stored at a storage condition of 40°C and 75% RH.

| **Formulation Details** | **Test Sample (Limits as per USP 29)** | |
|---|---|---|
| **%Impurities** | Initial | 3Mnths 40°C/75% RH |
| Fluvastatin N-ethyl Analog | ... | ... |
| Anti isomer | 0.074 | 0.122 |
| 3-keto 5 Hydroxy | ... | 0.09 |
| Impurity A (Hydroxy diene) | 0.097 | 0.222 |
| Fluvastatin short chain aldehyde | ... | ... |
| Fluvastatin t-butyl ester | ... | ... |
| 5 Keto 3 hydroxy | ... | 0.148 |
| Total Impurities | 0.227 | 0.745 |
| Any other unknown Impurity | ... | ... |
| Total Unknown Impurities | ... | ... |
| Highest Unknown Impurity | 0.042 | 0.132 |

The stability studies were performed at storage condition of 40°C and 75% RH and the parameters evaluated are listed in the Table 4.
The values of various parameters were found within the USP 29 limits.

## Claims

1. A color stable controlled release pharmaceutical dosage form comprising HMG CoA reductase inhibitor, rate controlling polymers and one or more pharmaceutical excipients wherein the granules are independent of particle size.

2. A color stable controlled release pharmaceutical dosage form as claimed in Claim 1 is free of alkalizing or buffering agents or combinations thereof.

3. The color stable controlled release pharmaceutical dosage form as claimed in claim 1 wherein the active substance is HMG CoA reductase inhibitor like Fluvastatin and/or its pharmaceutically acceptable salts.

4. The stable controlled release pharmaceutical dosage form as claimed in claim 1 wherein the rate controlling polymers is selected from hydroxypropyl methyl cellulose, hydroxypropyl cellulose and the like, alone or in combinations thereof.

5. The color stable controlled release pharmaceutical dosage form as claimed in claim 1 wherein the one or more pharmaceutical excipients is selected from diluents, disintegrants, lubricant, glident, binders, fillers, surfactant, solubilizers, and wetting agents and the like, alone or in combinations thereof.

6. A color stable controlled release pharmaceutical dosage form comprising Fluvastatin Sodium, hydroxypropyl methylcellulose polymer and hydroxypropyl cellulose, wherein the granules are independent of particle size.

7. The color stable controlled release pharmaceutical dosage form as claimed in Claim 6 can be prepared by direct compaction or dry compaction (slugging), wet granulation, non-aqueous granulation, melt granulation, and the like.

8. A color stable controlled release pharmaceutical dosage form as claimed in Claim 6 is free of alkalizing or buffering agents or combinations thereof.

9. A color stable controlled release pharmaceutical dosage form as claimed in Claim 6 wherein hydroxypropyl methylcellulose has a molecular weight of about 170000 to about 250000.

10. A color stable controlled release pharmaceutical dosage form as claimed in Claim 6 wherein hydroxypropyl cellulose has a molecular weight of about 80000 to about 1150000.

11. The color stable controlled release pharmaceutical dosage form as claimed in claim 6 wherein the ratio of the hydroxypropyl methylcellulose to hydroxy propylcellulose is from about 1:3.5 to 1:10.

12. A color stable controlled release pharmaceutical dosage form comprising granules comprising fluvastatin sodium and a hydroxypropyl cellulose polymer, wherein the granules are independent of particle size.

13. A color stable controlled release pharmaceutical dosage form as claimed in Claim 12 is free of alkalizing or buffering agents or combinations thereof.

14. A process of making a color stable controlled release dosage form comprising fluvastatin sodium and hydroxypropyl cellulose wherein the drug and excipients granulation is carried out under dry conditions.

15. A process of Claim 14 wherein the dry condition is direct compaction or dry compaction (slugging) carried under atmospheric condition.

16. The color stable controlled release pharmaceutical dosage form as claimed in Claim 14 can also be prepared by wet granulation, non-aqueous granulation, melt granulation, and the like.

17. The color stable controlled release pharmaceutical dosage form as claimed in any preceding claim wherein the rate controlling polymers is selected from the group comprising of carboxymethylcellulose, hydroxypropyl methylcellulose, hydroxypropyl ethyl cellulose, Hydroxypropyl cellulose, and polyethylene oxide and the like, or combinations thereof.

18. The color stable controlled release pharmaceutical dosage as claimed in any preceding claim wherein the coating composition comprises of hydroxypropyl cellulose, hydroxypropyl methylcellulose or combination thereof, glidant and colorants.

19. The color stable pharmaceutical dosage form as claimed in Claim 18 wherein the glidant of the coating is selected from the group comprising of talc, magnesium stearate, calcium stearate and combinations thereof.

20. The color stable pharmaceutical dosage form as claimed in Claim 18 wherein the colorant of the coating is selected from the group comprising of aluminum lakes, insoluble pigments, water-soluble dyes, titanium dioxide and talc.

21. A color stable controlled release pharmaceutical dosage form as claimed in claim 1 exhibits the following dissolution profile, when measured in type 1 dissolution apparatus, basket, at 50rpm, at a temperature of 37±0.5C, in 1000ml of water.
(i) not more than about 50% of fluvastatin is released after 2 hours of measurement in said apparatus
(ii) from about 50% to about 80% of fluvastatin is released after 4 hours of measurement in said apparatus
(iii) not less than about 80 % of fluvastatin is released after 8 hours of measurement in said apparatus; all weight percentages are based upon the total weight of the dosage form.
